# EUROPEAN PATENT APPLICATION

(11) **EP 0 786 471 A1**
(43) Date of publication of application: **30.07.1997**
(21) Application number: 97100319.9
(22) Date of filing: 10.01.1997
(51) Int. Cl.: C07K 11/02, C12P 21/02, C12N 1/14

(54) **Arthrichitin, a process for its production and its use**

(30) Priority: 23.01.1996 EP 96100859
(71) Applicant: HOECHST AKTIENGESELLSCHAFT, 65929 Frankfurt am Main (DE)
(72) Inventor: Roy, Kirity, Dr., Mulund (W), Bombay-400 082 (IN); Vijayakumar, Erra Koteswara Satya, Dr., Mulund (W), Bombay-400 082 (IN); Deshmukh, Sunil Kumar, Dr., Thane 400 601, Maharashta (IN); Chatterjee, Sugata, Dr., Bhopal 462 016 M.P. (IN); Ganguli, Bimal Naresh, Dr., Thane 400 607, Maharashtra (IN); Kogler, Herbert, Dr., 65779 Kelheim (DE); Fehlhaber, Hans-Wolfram, Dr., 24306 Plön (DE)

(57) **Abstract**

Arthrichitin, a compound of formula I 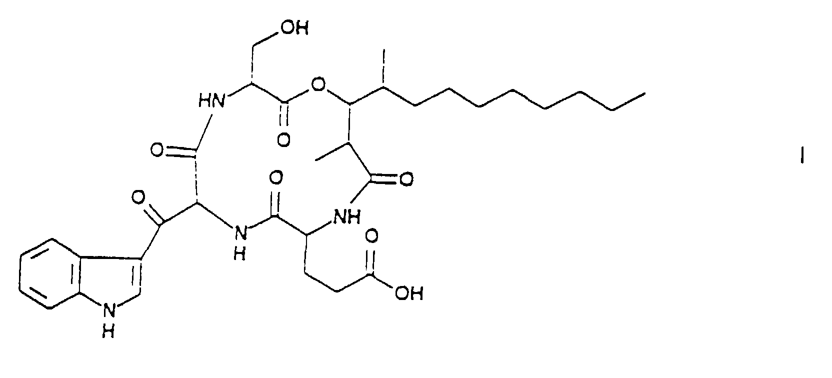 has a cell wall formation inhibitory activity.

## Description

This invention relates to Arthrichitin, a process for its production and its use. Arthrichitin is a compound of the following formula.

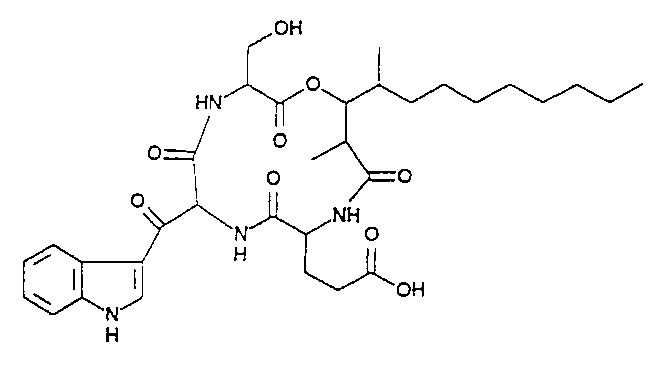

Arthrichitin has a hitherto unreported depsipeptide structure, formed by glutamic acid, serine, β-keto tryptophan and 2,4-dimethyl-3-hydroxydodecanoic acid. A chemical abstract literature search using search keys of the molecular formula and structure of the formula as shown above, established Arthrichitin to be a novel compound. No other compound represented the structural features of Arthrichitin. The instant invention relates also to the obvious chemical equivalents of Arthrichitin. Obvious chemical equivalents of Arthrichitin are compounds which can easily be produced with Arthrichitin or which can easily be converted to Arthrichitin such as acid addition salts or esters of Arthrichitin.

Another subject of the instant application is a process for the production of Arthrichitin by fermentation of the microorganism Y-90,3022, its mutants or variants.

The fungal culture Y-90,3022 for the production of Arthrichitin has been isolated from grass leaves collected from Mulund, Bombay, Maharashtra, India and has been identified as Arthrinium phaeospermum (Corda) Ellis and has been deposited on October 24, 1990 under the conditions of the Treaty of Budapest with Deutsche Sammlung von Mikroorganismen (accession number DSM No. 6245).

Isolation of the fungal culture is carried out using the direct soil plate method (Nature, 166, p 117, 1950) for isolation of fungi using the above leaves instead of soil sample.

The production of Arthrichitin comprises cultivating said fungal culture, its mutants or variants by fermentation under aerobic conditions in a nutrient medium of the kind herein described preferably at 24 to 30°C and pH 6.0 to 8.0 and isolating and purifying the Arthrichitin from the culture broth.

The aqueous nutrient medium consists of carbon sources such as glucose, sucrose, starch or dextrin. The preferred carbon source is starch. The nutrient medium also consists of sources of nitrogen such as tryptone, yeast extract, beef extract, cornsteep liquor, malt extract, soybean meal, peptone or inorganic materials such as ammonium salts. However, the preferred nitrogen source is tryptone or a combination of tryptone with one or more of other nitrogen sources. The nutrient medium also consists of nutrient inorganic salts such as sodium chloride, potassium hydrogen/dihydrogen phosphate or calcium carbonate. The nutrient medium also consists of trace elements such as salts of iron, manganese, copper, zinc, cobalt or such other heavy metals.

Preferably cultivation of the fungal culture Y- 90,3022, is carried out at 26°C (± 1°C) and pH of about 6.5.

The fermentation is preferably carried out for 66 to 115 hours when optimal yield of Arthrichitin of the present invention is found to have been obtained.

Fermentation carried out for about 90 hours under submerged condition in shake flasks in order to achieve the maximum activity is particularly preferred. The progress of fermentation and the formation of Arthrichitin of the present invention can be detected by the modified screening method of detecting the cell-wall active compounds [J. Antibiotics, 39(11), 1620-1622, 1986] using the Neurospora crassa strain SGF 18.

In the resulting culture broth, Arthrichitin is present in the culture fluid as well as in the mycelial cake and can be isolated using standard separation techniques. Thus, it can be recovered by extraction of the broth filtrate with a water immiscible solvent such as ethyl acetate, chloroform or butanol, or by hydrophobic interaction chromatography using polymeric resins such as ®Diaion HP-20 (Mitsubishi Chemical Industries Limited, Japan or ®Amberlite XAD (Rohm and Haas Industries, U.S.A.). The preferred method is adsorption over ®Diaion HP-20 followed by desorption of the antibiotic using eluants such as water, methanol, acetone or acetonitrile or combinations thereof. Concentration and lyophilization gives the crude antibiotic. Arthrichitin can also be recovered from the mycelium by extraction with water miscible solvents such as methanol, acetone or acetonitrile followed by concentration and hydrophobic interaction chromatography over polymeric resins such as Diaion HP-20 or Amberlite XAD or by extraction with water immiscible solvents such as ethyl acetate, chloroform or n-butanol. The preferred method is acetone extraction, concentration, dilution with water and adsorption over Diaion HP-20 followed by elution with water, methanol, acetonitrile, acetone or combinations thereof.

Arthrichitin can be further purified by using any of the following techniques: normal phase chromatography (alumina or silica gel; eluants such as ethyl acetate, chloroform, methanol or combinations thereof), reverse phase chromatography (dimethyl octadecylsilylsilica gel, also called RP-18 or dimethyl octasilylsilica gel, also called RP-8 (reverse-phase silica gel); eluants such as water, buffers viz. phosphate, acetate, citrate (pH 2-8) and organic solvents methanol, acetonitrile or acetone, or combinations of these solvents), gel permeation chromatography using resins such as ®Sephadex LH-20 (Pharmacia Chemical Industries, Sweden), using solvents such as methanol, chloroform or ethyl acetate or their combinations; or by counter-current chromatography using a biphasic eluant system made up of two or more solvents such as water and chloroform. These techniques may be used repeatedly or a combination of the different techniques may be used. The preferred method is chromatography over reverse-phase modified silica gel (RP-18).

Arthrichitin has an activity as cell wall inhibiting compound. It has been shown to be active in vivo against fungi, in particular against phytopathogenic fungi. A very good activity has been proven against Plasmopara viticola, Phytophthora infestans, Botrytis cinerea, Pyricularia oryzae, Erysiphe graminis, Septoria nodorum and Pseudocercosporella herpotrichoides.

Accordingly, another subject of the instant invention is the use of Arthrichitin as cell wall inhibiting compound, in particular as a compound against fungi. The compound can be used in different areas, it is particularly suitable in the field of plant protection.

An additional subject of the instant application are compositions containing Arthrichitin and the use of Arthrichitin for the production of such compositions.

In addition to Arthrichitin of the formula I, the compositions according to the invention may contain suitable formulation auxiliaries. The content of active compound of the formula I is in general between 1 and 95 % by weight.

They can be formulated in various manners, depending on the biological and/or physicochemical parameters. Suitable formulation possibilities are therefore: wettable powders (WP), emulsifiable concentrates (EC), aqueous dispersions on an oil or water basis (SC), suspoemulsions (SC), dusts (DP), dressing agents, granules in the form of water-dispersible granules (WG), ULV formulations, microcapsules, waxes or baits.

These individual formulation types are known in principle and are described, for example, in: Winnacker-Küchler, "Chemische Technologie*"*, Volume 7, C-Hauser Verlag München, 4th Ed., 1986; van Falkenberg, "Pesticides Formulations", Marcel Dekker N.Y., 2nd Ed., 1972 - 73; K. Martens, "Spray Drying Handbook", 3rd Ed., 1979, G. Goodwin Ltd. London.

The required formulation auxiliaries, such as inert materials, surfactants, solvents and other additives are also known and are described, for example, in: Watkins, "Handbook of Insecticide Dust Diluents and Carriers", 2nd Ed. , Darland Books, Caldwell N.J.; H. v. Olphen, "Introduction to Clay Colloid Chemistry*"*, 2nd Ed., J. Wiley & Sons, N.Y.; Marsden, "Solvents Guide", 2nd Ed., Interscience, N.Y., 1950; McCutcheon's "Detergents and Emulsifiers Annual", MC Publ. Corp., Ridgewood N.J.; Sisley and Wood, "Encyclopedia of Surface Active Agents", Chem. Publ. Co. Inc., N.Y.. 1964; Schönfeldt, "Grenzflächenaktive Äthylenoxidaddukte (Surface-Active Ethylene Oxide Adducts)*"*, Wiss. Verlagsges., Stuttgart, 1976; Winnacker-Küchler, "Chemische Technologie (Chemical Technology)*"*, Vol. 7, C. Hauser Verlag München, 4th Ed.81 1986.

Combinations with other pesticidally active substances, fertilizers and/or growth regulators can also be prepared on the basis of these formulations, for example in the form of a ready mix or as a tank mix.

Wettable powders are preparations which are uniformly dispersible in water and which, apart from the active compound, also contain wetting agents, for example polyoxyethylated alkylphenols, polyoxyethylated fatty alcohols, alkylsulfonates or alkylphenolsulfonates and dispersing agents, for example sodium ligninsulfonate, sodium 2,2'-dinaphthylmethane-6,6'-disulfonate, sodium dibutylnaphthalenesulfonate or alternatively sodium oleylmethyltauride in addition to a diluent or inert substance. Emulsifiable concentrates are prepared by dissolving the active compound in an organic solvent, for example butanol, cyclohexanone, dimethylformamide, xylene or alternatively high-boiling aromatics or hydrocarbons with the addition of one or more emulsifiers. Emulsifiers which can be used are, for example:

Calcium alkylarylsulfonates such as Ca dodecylbenzenesulfonate or nonionic emulsifiers such as fatty acid polyglycol esters, alkylaryl polyglycol ethers, fatty alcohol polyglycol ethers, propylene oxide/ethylene oxide, sorbitan fatty acid esters, polyoxyethylene sorbitan fatty acid esters or polyoxyethylene sorbitol esters.

Dusts are obtained by grinding the active compound with finely divided solid substances, for example talc, natural clays such as kaolin, bentonite, pyrophillite or di atomaceous earth. Granules can either be prepared by spraying the active compound onto adsorptive, granulated inert material or by applying active compound concentrates to the surface of carriers such as sand, kaolinites or granulated inert material by means of tackifiers, for example polyvinyl alcohol, sodium polyacrylate or alternatively mineral oils. Suitable active compounds can also be granulated in the manner customary for the preparation of fertilizer granules, if desired in a mixture with fertilizers.

The active compound concentration in wettable powders is, for example, about 10 to 90 % by weight, the remainder to 100 % by weight consisting of customary formulation constituents. In the case of emulsifiable concentrates, the active compound concentration can be about 5 to 80 % by weight. Dust-like formulations usually contain 5 to 20 % by weight.

In addition, said active compound formulations optionally contain the binders, wetting agents, dispersants, emulsifiers, penetrants, solvents, fillers or carriers which are customary in each case.

For use, the concentrates, present in commercially available form, are optionally diluted in a customary manner, for example by means of water in the case of wettable powders, emulsifiable concentrates, dispersions and in some cases also in the case of microgranules.

Preparations in the form of dusts and granulated preparations and also sprayable solutions are customarily not further diluted with other inert substances before use.

The application rate required varies, inter alia, with the external conditions such as temperature and humidity. It can vary within wide limits, for example between 0.005 and 10. 0 kg/ha or more of active substance, but it is preferably between 0.01 and 5 kg/ha.

### Example I: Isolation of culture Y-90,3022 from grass leaves

a. Composition of nutrient isolation medium
   - Peptone: 10 g
   - Glucose: 40 g
   - Agar: 20 g
   - Chloramphenicol: 0.05 g
   - pH: 6.5

   While preparing the medium chloramphenicol was added after dissolving the same in 10 ml of 95% ethanol and mixing thoroughly with the hot medium. The medium was autoclaved to a temperature of 121°C for 10 minutes. The pH prior to autoclaving was adjusted to 6.5.
b. Isolation :
   Grass leaves, which were collected from Mulund, Bombay, Maharashtra, India, were washed thoroughly with water and cut into small pieces and were placed in a glass petri plate (6*"* diameter).

The above medium was cooled to 45°C and poured into the above petri plate (ca. 80 ml) containing the small pieces of the leaves, swirled thoroughly and were allowed to settle. It was then incubated at room temperature (26°C ± 1°C) for 2 weeks. The plate was observed periodically and the culture Y-90,3022 was isolated from the growing microorganisms [Nature, 166, p117 (1950)].

### Example II: Maintenance of the culture Y - 90,3022

### Composition of maintenance medium

Culture No. Y-90,3022 was maintained on Sabouraud's glucose agar medium with the following modified composition :
- Glucose: 40 g
- Peptone: 10 g
- Lindane: 0.1 g
- Agar: 15 g
- Demineralized water: 1 litre
- pH: 6.5

The ingredients were dissolved by heating thoroughly, distribed in test tubes and then sterilized at 121°C for 20 minutes. The test tubes were first cooled and then allowed to solidify in a slanting position. The agar slants were inoculated with the growth of the culture Y-90,3022 by an inoculation needle and then incubated at 26°C (± 1°C) until a good growth was observed. The well grown cultures were stored in the refrigerator.

### Example III: Fermentation of culture Y-90,3022 in shake flasks

### Composition of seed culture medium

- Soluble starch: 15 g
- Soybean meal: 15 g
- Glucose: 5 g
- CaCO₃: 2 g
- NaCl: 5 g
- Yeast extract: 2 g
- Cornsteep liquor: 1 g
- ZnSO₄·7H₂O: 0.22 mg
- CaCl₂: 0.55 mg
- MnCl₂·4H₂O: 0.5 mg
- FeSO₄·5H₂O: 0.16 mg
- CuSO₄·5H₂O: 0.16 mg
- CoCl₂·6H₂O: 0.16 mg
- Demineralised water: 1 litre
- pH prior to autoclaving: 6.5

The above medium was distributed in 100 ml amounts in 500 ml wide mouth Erlenmeyer flasks and autoclaved at 121°C for 20 mins. The flasks were cooled and then inoculated with few loopfuls of the above mentioned well sporulated culture of Example II and shaken at 200 rpm for 48 hours at 26°C (± 1°C) . This was used as the seed culture for inoculating the production medium of the same composition as mentioned for seed medium.

The production medium was distributed in 200 ml amounts in 1 litre Erlenmeyer flasks and autoclaved at 121°C for 20 minutes. The flasks were cooled and then inoculated with the above mentioned seed cultures (1% v/v). The fermentation was carried out on a rotary shaker at 200 rpm and at a temperature of 26°C (± 1°C) for 96 hours.

The production of Arthrichitin was monitored by using the modified screening method of detecting cell-wall active compounds [J. Antibiot., 39 (11), 1620-22, 1986] using the Neurospora crassa strain SGF 18 and growing it in a medium containing the following nutrients : soluble starch 1.5%; Yeast extract 0.4%; K₂HPO₄ 0.1 %; MgSO4.7H2O 0.05%; D-Sorbitol 7.5% (Osmotic stabilizer); L-Sorbose 2.5% (Glucan synthesis inhibitor) and agar 1.5%.

### Example IV: Fermentation of culture Y-90,3022 in fermentors

### Stage I: Preparation of seed culture

In shake flasks : The seed culture was prepared in 1 litre wide mouth Erlenmeyer flasks (with 150 ml medium) as mentioned in Example III, pooled and transferred into a 5-litre sterile aspirator bottle aseptically. This was used to inoculate the 150-litre fermentor.

### Stage II: Fermentation

One hundred litres of the production medium (as mentioned in Example III) were taken in a 150-litre fermentor with presterilization pH adjusted to 6.5 and 0.04% ®Desmophen added as an antifoam. The medium was sterilized at 121-122°C for 32 minutes at 1.2 kg/cm² steam pressure. pH after sterilization was 6.0. After cooling to 26°C (± 1°C), it was inoculated with 1-3% (v/v) of seed culture under aseptic conditions. The fermentation was carried out for 69 hours with an agitation of 100-120 rpm and aeration of 100 lpm. The production of Arthrichitin was monitored as mentioned in Example III. The broth was harvested, centrifuged and processed further as described in Example V.

### Example V: Isolation and purification of Arthrichitin

Approximately 100 litres of the culture broth were harvested after 69 hours and separated from the mycelium (6.5 kg) by centrifugation. Arthrichitin was found to be present in the culture filtrate as well as in the mycelium. The culture filtrate was passed through a column of 4 litres of Diaion HP-20 and the column was washed with demineralized water (40 litres) and then eluted with methanol (10 litres). The fractions were collected in 1 litre size and monitored by its activity against Neurospora crassa strain SGF 18. Arthrichitin eluted out in methanol and the active fractions were combined, concentrated under reduced pressure of 10 - 100 mm at 30-40°C and lyophilized to get 17 g of crude Arthrichitin. The mycelium was extracted with 2 x 40 litres of acetone, the acetone removed under reduced pressure of 10 - 100 mm at 30-40°C and the residual aqueous material was diluted with water to 10 litres. This solution was passed through a column of 500 ml of Diaion HP-20. The column was washed with demineralized water (5 litres) and then eluted with methanol (7 litres). The eluates were collected in fractions of 1 litre and the presence of Arthrichitin in each fraction was monitored by its activity against Neurospora crassa strain SGF 18. The active methanol eluates containing Arthrichitin were combined and concentrated under reduced pressure of 10 - 100 mm at 30-40°C to about 100 ml. The concentrate was lyophilyzed to give 8 g of crude Arthrichitin as an off-white powder. This was combined with the crude material obtained from culture filtrate and subjected to medium pressure liquid chromatography (MPLC) on RP-18 (reverse-phase silica gel) packed in a 7.4 cm x 51.4 cm glass column. The column was developed in 0.02 M aqueous sodium phosphate buffer of pH 7.0 (2.5 litres) and then eluted with acetonitrile-phosphate buffer mixture where the acetonitrile concentration was increased in steps of 5%. Thus elution was done with 10% (2 litres), 15% (2 litres). 25% (4.5 litres), 30% (1.5 litres) and then with 50% (1 litre) and acetonitrile (1 litre). A flow rate of 18 ml per minute was maintained. The eluates were collected in fractions of 500 ml and the presence of Arthrichitin was monitored by UV detection at 220 nm and also by checking each fraction for their activity against Neurospora crassa SGF 18. Arthrichitin eluted out in 25-30% acetonitrile in phosphate buffer system which was concentrated under reduced pressure of 10 to 100 mm at 30-40°C to about 3 litres of an aqueous solution. This solution was desalted by passing through a column of Diaion HP-20 (500 ml). The column was washed with demineralized water till the washings were phosphate free and eluted with methanol (3 litres). The fractions were collected in 300 ml size. The active fractions were combined and concentrated under reduced pressure of 10 to 100 mm at 30-40°C to yield 4.2 g of pure Arthrichitin.

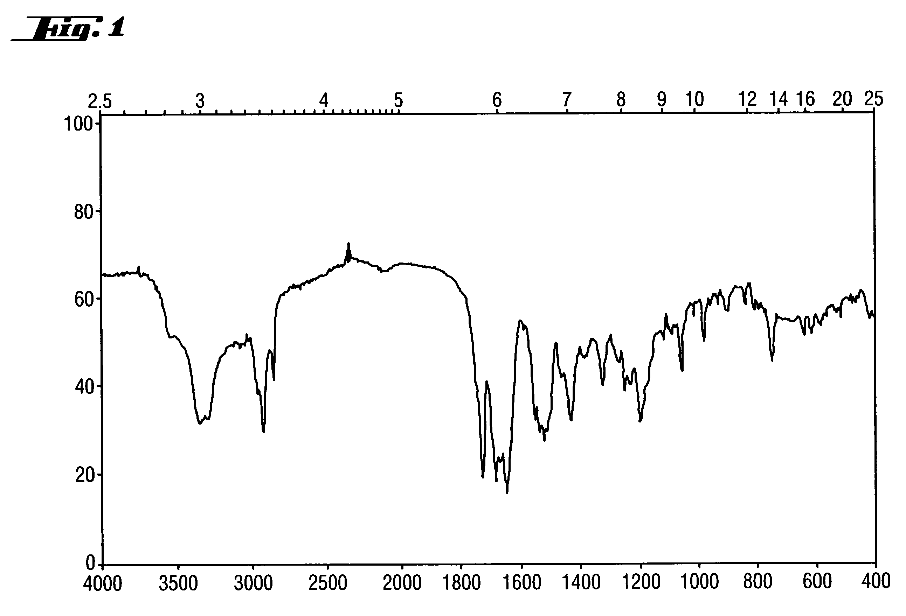

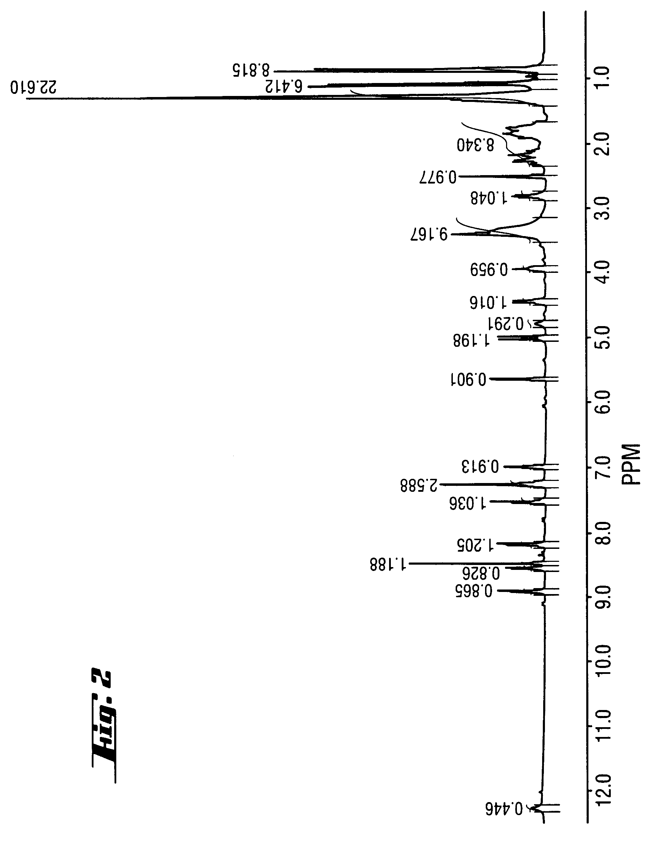

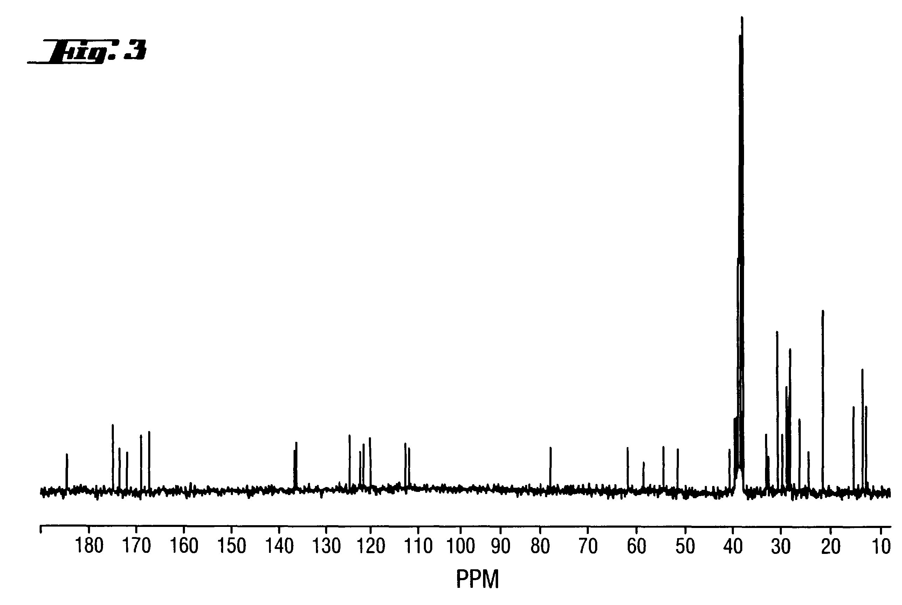

The physico-chemical properties of Arthrichitin are as follows :
- Appearance: : Colorless solid
- Solubility: : Soluble in methanol and dimethylsulfoxide; insoluble in petroleum ether, chloroform, ethylacetate and water.
- Molecular weight: : 642 (FAB MS) (Matrix : 3-nitrobenzyl alcohol)
- Molecular formula: : C₃₃H₄₆N₄O₉ (calculated : m/z 642.2262;
[observed : m/z 643.3341 (M + H)⁺ (HR FAB-MS; matrix : MeOH/NBA; internal reference : polyethylene glycol 600); calculated for C₃₃H₄₇N₄O₉ : 643.3345]
- Melting point: : 245°C
- [α]_{D}: : + 24.12°C (*c* 0.475, MeOH)
- HPLC retention time: : 5.4 minutes
[4 x (30 + 100) mm 10µ ODS ®Hypersil column; mobile phase : 35% acetonitrile in 0.02 M aqueous sodium phosphate buffer, pH 7.0; flow rate : 1 ml/min.; UV detection : 220 nm]
- UV λₘₐₓ (MeOH): : 247, 266, 310 nm; no shift with acid and alkali
- IR (KBr): : Figure 1
- ¹H NMR (300 MHz, DMSO-d₆): : Figure 2
- ¹³C NMR (75 MHz, DMSO-d₆): : Figure 3

On the basis of the above physicochemical properties and additional 2D-NMR experiments as well as degradative studies, the structure of Arthrichitin was deduced as a depsipeptide of the formula I formed by four different units of glutamic acid, serine, β-ketotryptophan and 2,4-dimethyl-3-hydroxy- dodecanoic acid. This structure is not reported in the literature.

## Claims

1. Arthrichitin, a compound of formula I 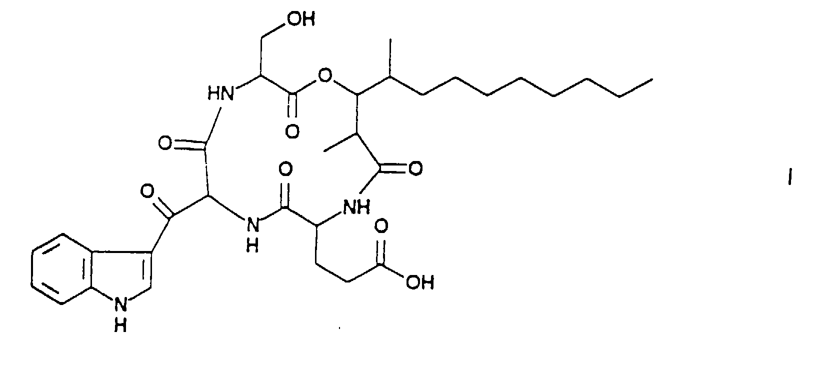 as well as the obvious chemical equivalents thereof.

2. A process for the preparation of Arthrichitin as claimed in claim 1, which comprises Arthrichitin phaespermum Y-90,3022 (DSM 6245), the mutants and/or variants thereof, being cultivated under aerobic conditions in a nutrient medium containing sources of carbon and of nitrogen, inorganic nutrient salts and trace elements, and the compound being isolated and purified from the culture broth in a customary manner.

3. The process as claimed in claim 2, wherein the cultivation is carried out at temperatures from about 24 to 30°C and at a pH between about 6 and 8.

4. The process as claimed in claims 2 and 3, wherein the cultivation is carried out at 26°C (± 1°C) and a pH about 6.5.

5. The process as claimed in one or more of claims 2 to 4, wherein the fermentation is carried out as submerged fermentation.

6. The use of Arthrichitin for the inhibition of cell wall formation.

7. The use of Arthrichitin for the production of a composition for the inhibition of cell wall formation.

8. A composition having an active amount of Arthrichitin, optionally together with suitable formulation auxiliaries.

9. A process for the production of a composition as claimed in claim 8, wherein Arthrichitin is, optionally together with suitable auxiliaries, brought into a form suitable for administration.

10. The microorganism Arthrinium phaeosporum (DSM 6245).
